# FASCICULE DE BREVET EUROPEEN

(11) **EP 0 799 242 B1**
(45) Date de publication et mention de la délivrance du brevet: **18.04.2007**
(21) Numéro de dépôt: 96934924.0
(22) Date de dépôt: 16.10.1996
(51) Int. Cl.: C07K 14/415, A61K 38/16, A61Q 5/02, A61Q 19/00, A61Q 19/08, A61K 8/64

(54) **COMPLEXES AMPHIPHILES, PROCEDE POUR LEUR PREPARATION ET COMPOSITIONS EN RENFERMANT**
AMPHIPHILE KOMPLEXE, METHODEN ZU DEREN HERSTELLUNG UND DIESE ENTHALTENDE VERBINDUNGEN
AMPHIPHILIC COMPLEXES, METHOD FOR PREPARING SAME AND COMPOSITIONS CONTAINING SUCH COMPLEXES

(30) Priorité: 17.10.1995 FR 9512137
(43) Date de publication de la demande: 08.10.1997
(73) Titulaire: ENGELHARD LYON, 69007 Lyon (FR)
(72) Inventeur: PERRIER, Eric, 38200 Vienne (FR); HUC, Alain, 69110 Sainte-Foy-lès-Lyon (FR); ANTONI, Danielle, 69390 Vernaison (FR); ROUSSEL, Coralie, 34470 Perols (FR); PINAL, Michel, 34080 Montpellier (FR); GRAILLE, Jean, 34750 Villeneuve-les-Maguelonne (FR)
(74) Mandataire: Portal, Gérard
(86) Numéro de dépôt international: PCT/FR1996/001620
(87) Numéro de publication internationale: WO 1997/014713

(56) Documents cités:
- EP-A- 0 283 601
- EP-A- 0 298 796
- EP-A- 0 417 619
- WO-A-92/13006
- WO-A-93/22370
- DE-A- 3 422 496
- US-A- 4 234 475

## Description

La présente invention a pour premier objet des complexes amphiphiles (hydrolipidiques) et plus précisément des protéines sur lesquelles ont été greffées des chaînes grasses. On peut qualifier lesdits complexes de protéines lipophilisées. La présente invention a également pour objets des compositions notamment cosmétiques, pharmaceutiques ou alimentaires renfermant de tels complexes et des procédés pour la préparation desdits complexes.

La peau peut être considérée comme un organe qui sépare et protège le corps humain de son environnement. Cet effet de barrière contre les agressions extérieures est capital pour que les tissus internes puissent exercer convenablement leur fonction. Les agressions extérieures sont en effet multiples : agressions lumineuses (UVA, UVB, infra-rouges) qui provoquent radicaux libres et fragmentation des constituants de la peau, agressions physiques ou mécaniques (frottements, variations de température et d'hygrométrie ...) qui provoquent des inflammations, agressions chimiques (pollution de l'air, de l'eau, contact avec des éléments irritants ou immunogènes), agressions microbiologiques (bactéries, virus, champignons ...). Afin de réagir à ces différentes agressions, la peau dispose d'un certain nombre de cellules spécialisées, formant parfois des structures extrêmement bien caractérisées. C'est le cas des cornéocytes qui, différenciés à partir des kératinocytes, forment une structure appelée Stratum corneum, qui est spécialisée dans la protection des zones plus internes de la peau. Cette structure cornée superficielle est la première protection vis-à-vis des agressions extérieures.

L'utilisation de produits cosmétiques et notamment de produits d'hydratation se heurte également à cette barrière naturelle :
- de par leur petite taille, des molécules hydrophiles de petite masse moléculaire comme l'urée, l'acide lactique, les acides aminés, peuvent pénétrer via le Stratum corneum, jusque dans des couches plus profondes des tissus cutanés. L'effet cosmétique obtenu est un effet hydratant des couches profondes de l'épiderme et du derme, effet généralement relativement court;
- par contre, des molécules de plus haute masse moléculaire comme les protéines par exemple ne peuvent pas franchir cette barrière. En effet, le Stratum corneum est principalement constitué de lipides (sa teneur en lipides est proche de 80 % en poids), ce qui lui donne un caractère particulièrement hydrophobe, totalement incompatible avec le caractère hydrophile de la plupart des protéines utilisées dans le domaine cosmétologique. Dans ce cas, l'effet cosmétique obtenu est un effet filmogène, parfois intéressant pour l'obtention de textures ou de "touchers cosmétiques" particuliers, mais qui reste totalement et exclusivement superficiel.

Ainsi, et par voie de conséquence, les molécules hydrophiles utilisées à ce jour en cosmétique sont repoussées par cette structure hydrophobe et soit restent en surface soit pénétrent très profondément dans le derme. De ce fait, la couche cornée et les couches supérieures de l'épiderme ne subissent pratiquement aucune influence des substances actives et notamment hydratantes utilisées à ce jour en cosmétique. Or, l'impression de sécheresse de la peau provient du Stratum corneum et des couches supérieures de l'épiderme. Il est donc capital de parvenir à hydrater efficacement cette structure et plus généralement de rendre accessible à diverses entités hydrophiles ladite structure.

La Demanderesse, dans le cadre de la présente invention, a abordé ce problème technique de l'hydratation de la peau et plus généralement celui de l'optimisation de l'expression de l'activité de molécules du type protéine au niveau du Stratum corneum. Pour résoudre ledit problème technique, elle propose de modifier le caractère physico-chimique desdites molécules et d'en modifier ainsi le comportement. Elle propose en fait de générer des complexes amphiphiles en greffant des chaînes grasses sur lesdites molécules. Les pénétrations trans-épidermiques de tels complexes sont différentes de celles des molécules non complexées. Leur stabilisation dans les couches supérieures de l'épiderme ainsi que sur la fibre capillaire (cheveux) a été mise en évidence. Par ailleurs, on a observé, des propriétés cosmétiques voire thérapeutiques desdits complexes, fort intéressantes et inattendues.

Il a été décrit, dans la demande de brevet FR-A-2 671 725 des complexes polyoses-acides gras qui présentent des propriétés hydratantes et émulsionnantes. Ces complexes sont obtenus en faisant réagir, en milieu aqueux, à la température ambiante, des acides gras sous forme réactive avec des polyoses. Lesdits polyoses peuvent intervenir sous forme impure et notamment en mélange avec des protéines. Toutefois, dans ce document, il n'est fait aucune mention d'un complexe "binaire" protéine-acide gras et des quelconques propriétés intéressantes qu'il pourrait présenter ... En tout état de cause, les polysaccharides (polyoses) présentant un pouvoir gélifiant bien supérieur à celui des protéines, on ne pouvait s'attendre à obtenir des complexes hydratants et émulsionnants en lipophilisant de telles protéines. Tel est pourtant l'un des résultats obtenus dans le cadre de la présente invention ...

Il a également été décrit :
- dans le brevet US-A-4,234,475 un procédé de préparation d'émulsionnants qui consiste à faire réagir, à des températures supérieures à 200°C, une protéine et un acide, notamment un acide gras. A de telles températures, on ne peut éviter la dégradation de chacun des réactifs, et notamment celle de la protéine (dénaturée et/ou hydrolysée en peptides), dont les propriétés sont en conséquence inéluctablement altérées ;
- dans la demande WO-A-93 22370 des dérivés de l'acide undécylénique obtenus en faisant réagir ledit acide, sous forme réactive, en milieu aqueux, à la température ambiante, avec une macromolécule organique hydrophile possédant des groupements alcools primaires et/ou amines primaires et notamment avec une protéine. Lesdits dérivés, très faiblement odorants, ont conservé des propriétés anti-fongique et anti-bactérienne. On a surtout cherché, par leur biais, à optimiser l'expression des activités de l'acide undécylènique.

Par ailleurs :
- la demande DE-A-34 22 496 décrit une composition alcoolique désinfectante de la peau. Ladite composition renferme un hydrolysat de protéines, en fait un mélange d'aminoacides,
- la demande EP-A-0 417 619 propose, à titre de détergent manifestant une agressivité moindre vis-à-vis de la peau et des muqueuses, les produits de condensation résultant de la réaction chimique entre :

- un hydrolysat de protéines dont la masse moléculaire moyenne est comprise entre 3 000 et 7 000 ; et
- un acide gras en C₁₂-C₁₈;
   ladite réaction chimique étant mise en oeuvre à un pH compris entre 7 et 12 et le rapport molaire protéine(s)/acide(s) gras variant de 1/0,5 à 1/3;
- la demande EP-A-0 283 601 décrit des dérivés d'élastine préparés à partir d'élastine hydrolysée. Lesdits dérivés résultent d'un couplage chimique entre ladite élastine hydrolysée (non native) et un anhydride d'acide gras ; ledit acide gras intervenant, par rapport à la protéine (élastine hydrolysée) en un rapport pondéral très inférieur à 1.

Lesdits produits de condensation selon EP-A-0 417 619 et dérivés d'élastine selon EP-A-0 283 601 ne sont pas des complexes au sens de l'invention. Lesdits complexes de l'invention sont toujours élaborés en présence d'un excès d'acide gras et sont élaborés avec des protéines natives. Ceci est explicité ci-après.

La Demanderesse propose en fait l'utilisation de nouveaux complexes amphiphiles ou hydrolipidiques - complexes protéine(s)/chaîne(s) grasse(s) - qui, comme indiqué ci-dessus, présentent des propriétés cosmétiques voire thérapeutiques fort intéressantes et relativement inattendues, comme défini dans les revendications.

On précise ici que, dans le présent texte - dans le contexte de l'invention - on emploie le terme protéine pour désigner une "réelle" protéine

Lesdits complexes de l'invention sont, de façon caractéristique, obtenus à l'issue de la réaction, mise en oeuvre à une température comprise entre la température ambiante et 80°C, entre :
- d'une part, une (ou plusieurs) protéine(s), dont la masse moléculaire moyenne est supérieure ou égale à 10.000 Daltons; et
- d'autre part, une (ou plusieurs) chaîne(s) grasse(s), dont le nombre de carbone est compris entre 4 et 30, choisie(s) parmi les acides gras, les alcools gras, les amines grasses et leurs dérivés, à l'exclusion de l'acide undécyclénique, le rapport pondéral des réactifs [protéine(s)/chaîne(s) grasse(s)] variant de 1/1 à 1/10 et avantageusement de 1/3 à 1/5.

La réaction mise en jeu pour le couplage et/ou greffage des réactifs peut être de type chimique ou enzymatique. Ceci sera précisé plus avant dans le présent texte. En tout état de cause, elle est mise en oeuvre à une température bien inférieure à 200°C, inférieure à 100°C. On souhaite minimiser voire éviter toute dégradation des réactifs et notamment des protéines intervenantes.

Ladite réaction est mise en oeuvre avec deux types de réactifs : d'une part, au moins une protéine, d'autre part au moins une chaîne grasse. Lesdites chaînes grasses consistent aussi bien en des acides gras que des alcools gras ou amines grasses (ou en les dérivés desdits acides, alcools et amines).

On peut d'ores et déjà insister sur la multiplicité et la variété des complexes de l'invention et donc des propriétés qu'ils sont susceptibles de présenter; ces dernières dépendant de la nature des réactifs (protéine(s) et chaîne(s) grasse(s) - qui, comme indiqué ci-dessus, présentent des propriétés cosmétiques voire thérapeutiques fort intéressantes et relativement inattendues.

Plus précisément, l'invention concerne une composition cosmétique renfermant de tels complexes, telle que définie à la revendication 1.

Selon un deuxième aspect, la présente invention concerne aussi une composition pharmaceutique renfermant un tel complexe telle que définie à la revendication 2.

On précise ici que, dans le présent texte -dans le contexte de l'invention- on emploie le terme protéine pour désigner aussi bien une "réelle" protéine qu'un polypeptide (obtenu éventuellement par synthèse).

Lesdits complexes de l'invention sont, de façon caractéristique, obtenus à l'issue de la réaction, mise en oeuvre à une température comprise entre la température ambiante et 80°C, entre:
- d'une part, une (ou plusieurs) protéine(s), dont la masse moléculaire moyenne est supérieure à 10 000 Dalton;
   et
- d'autre part, une (ou plusieurs) chaîne(s) grasse(s), dont le nombre de carbone est compris entre 4 et 30, choisie(s) parmi les acides gras, les alcools gras, les amines grasses et leurs dérivés, à l'exclusion de l'acide undécylénique, le rapport pondéral des réactifs protéine(s)/chaîne(s) grasse(s) variant de 1/1 à 1/10 et avantageusement de 1/3 à 1/5.

La réaction mise en jeu pour le couplage et/ou greffage des réactifs peut être de type chimique ou enzymatique. Ceci sera précisé plus avant dans le présent texte. En tout état de cause, elle est mise en oeuvre à une température bien inférieure à 200°C, inférieure à 100°C. On souhaite minimiser voire éviter toute dégradation des réactifs et notamment des protéines intervenantes.

Ladite réaction est mise en oeuvre avec deux types de réactifs : d'une part, au moins une protéine, d'autre part au moins une chaîne grasse. Lesdites chaînes grasses consistent aussi bien en des acides gras que des alcools gras ou amines grasses (ou en les dérivés desdits acides, alcools et amines).

On peut d'ores et déjà insister sur la multiplicité et la variété des complexes de l'invention et donc des propriétés qu'ils sont susceptibles de présenter; ces dernières dépendant de la nature des réactifs (protéine(s) et chaîne(s) grasse(s)) intervenants et de leurs caractéristiques intrinsèques (par exemple, nature de la protéine intervenant, pureté de celle-ci, poids moléculaire de celle-ci).

On précise ci-après, chacun des deux types de réactifs.

Les complexes de l'invention sont des complexes de protéines et de chaînes grasses. Sont exclus du cadre de ladite invention les complexes obtenus à partir d'acides aminés, que ceux-ci soient purifiés ou obtenus en mélange lors de l'hydrolyse d'une protéine ainsi que les complexes obtenus à partir de peptides ne renfermant que 2 à 5 acides aminés dans leur structure. Les protéines susceptibles d'intervenir dans la structure des complexes de l'invention ont une masse moléculaire moyenne égale ou supérieure a 10 000 Daltons. Ils consistent en un enchaînement d'acides aminés liés entre eux par des liaisons amides, qui présente des fonctions amines et/ou acides et/ou alcools pendantes.

Généralement, leur masse moléculaire moyenne est inférieure à 1 000 000 Daltons. Il n'est toutefois nullement exclu de préparer des complexes de l'invention avec des protéines d'une masse moléculaire moyenne supérieure. Avantageusement, les complexes de l'invention sont préparés à partir de protéines, dont la masse moléculaire moyenne est comprise entre 10000 et 1 000 000 Daltons. De façon encore plus avantageuse, on fait intervenir des protéines dont la masse moléculaire moyenne est comprise entre 20 000 et 300 000 Daltons.

En tout état de cause, les protéines intervenantes peuvent être obtenues par une extraction ne détruisant pas leur structure et/ou ne diminuant pas leur masse moléculaire.

Lesdites protéines intervenantes sont issues de plantes (blé, algues unicellulaires ou multicellulaires, maïs, pois, lupin ...) et dans ce cas, elles peuvent être extraites de graines, de fleurs, de fruits, d'écorces, de gommes, etc ... ; citons pour exemple les protéines de blé, de maïs, de coton, de lupin, de pois, de fève, d'amande, de féverole, de soja, de tournesol, de luzerne, d'avoine, etc ...

On prépare avantageusement des complexes de l'invention avec des protéines de soja, de blé, d'avoine ou d'amande.

Pour ce qui concerne le deuxième type de réactifs, il s'agit comme déjà précisé, de chaînes grasses comportant de 4 à 30 atomes de carbone. Avantageusement, les chaînes grasses intervenantes comportent de 6 à 20 atomes de carbone. Elles peuvent être saturées ou insaturées, linéaires, branchées ou cycliques. Elles présentent évidemment des fonctions acide et/ou alcool et/ou amine mais il n'est nullement exclu qu'elles présentent d'autres fonctions chimiques dans leur structure qui interviennent ou n'interviennent pas dans la préparation des complexes de l'invention.

Lesdites chaînes grasses peuvent notamment être choisies parmi les acides gras heptanoïque, octanoïque, décanoïque, laurique, myristique, palmitique, stéarique, ricinoléique, oléique, linoléique, linolénique; les alcools gras et amines grasses correspondants; les dérivés desdits acides gras, alcools gras et amines grasses ; et leurs mélanges.

On prépare avantageusement des complexes de l'invention :
- avec les acides laurique, stéarique ou palmitique et notamment les acides stéarique et palmitique en mélange;
- avec de la laurylamine ou de l'hexadécylamine;
- avec du décylalcool.

On a vu que lesdites chaînes grasses consistent en des acides gras, des alcools gras ou des amines grasses (ou leurs dérivés). Pour la préparation des complexes de l'invention par voie chimique, lesdits acides gras interviennent éventuellement sous des formes réactives (plus réactives), et notamment sous la forme d'halogénures (chlorures, bromures, iodures ...), d'anhydrides ou de dérivés d'anhydrides.

Au sein de la structure des complexes de l'invention, le rapport pondéral [protéine(s)]/[chaîne(s) grasse(s)] varie de 1/1 à 1/10 et préférentiellement de 1/3 à 1/5.

On fait, en fait, toujours réagir la (les) protéine(s) avec un excès plus ou moins large de chaînes grasse(s) dans le but de créer des liaisons covalentes mais aussi des liaisons de type ionique, hydrogène, Van der Waals.

Par ailleurs, généralement, à l'issue de la réaction, on ne récupère pas les chaînes grasses qui n'ont pas réagi, qui ne sont pas liées à la protéine, on ne cherche pas à isoler des complexes "binaires" du type : protéine(s)-chaînes grasses purs. Ainsi, les complexes de l'invention consistent-ils généralement en des complexes "binaires" du type indiqué ci-dessus en mélange avec des chaînes grasses non liées ; en d'autres termes, en le produit de la réaction de couplage en mélange avec les chaînes grasses qui n'ont pas réagi (qui ne sont pas couplées). L'utilisation desdits complexes constitue le premier objet de la présente invention. Les compositions, notamment cosmétiques, pharmaceutiques ou alimentaires, en renfermant constituent le second objet de ladite invention.

Lesdites compositions renferment généralement de 0,01 à 40 % en poids de tel(s) complexe(s) et avantageusement de 0,1 à 10 % en poids.

Plus précisément, font partie intégrante de la présente invention, les compositions notamment cosmétiques, pharmaceutiques ou alimentaires qui renferment, à titre d'ingrédient actif, au moins une protéine ; ladite protéine intervenant, au moins en partie (voire en totalité) sous la forme d'un complexe tel que décrit ci-dessus. Pour l'élaboration desdites compositions on peut utiliser ledit complexe, purifié (isolé du milieu réactionnel dans lequel il a été synthétisé) ou en mélange avec l'un et/ou l'autre des réactifs qui sont intervenus dans sa synthèse. Selon cette seconde variante, on utilise avantageusement le milieu réactionnel (à l'issue de la réaction) renfermant ledit complexe et les réactifs n'ayant pas réagi (principalement des chaînes grasses dans la mesure où elles interviennent en excès).

Font également partie de l'invention, les compositions où lesdits complexes interviennent à titre d'agents émulsionnants.

En tout état de cause, on a constaté, de manière surprenante, que les complexes de l'invention présentent des propriétés hydratantes et émulsionnantes. Ceci est relativement inattendu dans la mesure où l'homme du métier n'ignore pas que les protéines ont un pouvoir de piéger l'eau bien inférieur à celui des polysaccharides et où ledit pouvoir, relativement faible dans l'absolu, aurait dû être affecté par la lipophilisation desdites protéines.

Outre ces propriétés hydratantes et émulsionnantes - relativement inattendues - les complexes de l'invention ont montré d'autres propriétés - totalement inattendues.

C'est ainsi qu'une protéine de blé soluble, possédant une masse moléculaire moyenne de 100 000 Daltons, sur laquelle des chaînes d'acides stéarique et palmitique ont été greffées, présente des propriétés de restructuration cutanée extrêmement fortes, ce qui permet d'envisager l'utilisation de cette protéine lipophilisée (complexe au sens de l'invention) dans des applications où une destructuration de l'épiderme est observée (agressions physico-chimiques ou vieillissement cutané ...).

De même, une protéine d'amande soluble, possédant une masse moléculaire moyenne de 30 000 Daltons, sur laquelle des chaînes d'acides stéarique et palmitique ont été greffées, présente la propriété de calmer des érythèmes solaires modérés à forts, ce qui permet d'envisager l'utilisation de cette protéine lipophilisée (complexe au sens de l'invention) dans des formulations solaires ou après solaires.

De même, une protéine de laminaire insoluble, possédant une masse moléculaire moyenne de 10000 Daltons, sur laquelle des chaînes d'acide caprylique ont été greffées, présente la propriété d'inhiber un certain nombre de micro-organismes, ce qui permet d'envisager l'utilisation de cette protéine lipophilisée (complexe au sens de l'invention) dans des applications où la destruction des micro-organismes est envisagée (effets anti-acné, effets anti-pelliculaire, effets anti-odeurs corporelles, conservateur naturel ...).

On a précédemment insisté sur la diversité des complexes de l'invention. On saisit ici tout l'intérêt d'une telle diversité.

Par ailleurs, on rappelle ici que les propriétés des complexes de l'invention, qu'elles soient plus ou moins inattendues, s'expriment au niveau souhaité, au niveau du Stratum corneum, de par leur lipophilisation.

Les compositions de l'invention consistent donc essentiellement en des compositions cosmétiques. Il n'est pas exclu qu'ils s'agissent de compositions thérapeutiques, alimentaires ou diététiques particulièrement performantes au niveau des muqueuses.

Selon son troisième objet, l'invention concerne un procédé pour la préparation des complexes amphiphiles (hydrolipidiques) décrits ci-dessus. Ledit procédé comprend, de façon caractéristique, la réaction, à une température comprise entre la température ambiante et 80 °C, si nécessaire en milieu aqueux ou solvant, entre au moins une protéine du type précité et au moins une chaîne grasse du type précité, lesdits réactifs intervenants dans un rapport pondéral [protéine(s)/chaine(s) grasse(s)] compris entre 1/1 et 1/10, avantageusement entre 1/3 et 1/5, tel que défini à la revendication 20. Ladite réaction peut être qualifiée de réaction de greffage ou plus exactement de couplage (dans la mesure où elle ne génère pas seulement des liaisons covalentes entre les réactifs).

Ladite réaction est mise en oeuvre à relativement basse température. On minimise ainsi la dégradation des protéines réactives. Elle fait intervenir ou non un solvant, milieu aqueux ou solvant organique. On peut se dispenser de l'intervention d'un tel solvant si, à la température de réaction les réactifs sont liquides.

A l'issue de la réaction, les complexes "binaires" ne sont généralement pas isolés. Ils se trouvent alors en mélange principalement avec des chaînes grasses qui n'ont pas réagi.

On souhaite généralement ajuster le pH des complexes obtenus, afin de le rendre compatible avec des applications, notamment cosmétiques, ultérieures. On l'ajuste à des valeurs comprises entre 2 et 10 et plus particulièrement comprises entre 5 et 7. A cette fin, on utilise des agents neutralisants choisis parmi :
- les bases minérales (telles KOH, NaOH, Ca(OH)₂...);
- les bases métalliques (sous forme d'hydroxyde, de carbonate ...);
- les bases organiques (tampons citrates, phosphates, borates, acétates, TRIS ... ; amines ou alkylamines en C₁-C₆ : triéthanolamine, aminométhylpropane ...).

Après la réalisation des complexes dont les pH ont été si nécessaire ajustés à des pH compatibles avec leur utilisation ultérieure (ledit pH est avantageusement ajusté par dispersion desdits complexes en phase aqueuse), il est possible de les sécher par atomisation, lyophilisation, déshydratation sous vide ... Lesdits complexes séchés, ou directement obtenus sans eau, peuvent être alors mis en forme, notamment sous la forme de copeaux.

La réaction de couplage mise en oeuvre peut être réalisée par voie chimique ou enzymatique. La voie enzymatique est, dans le contexte de la présente invention, totalement originale.

Selon ladite voie chimique, on peut :
+ faire réagir les chaînes grasses - acides gras, alcools gras, amines grasses - dans des conditions classiques de la synthèse peptidique; i.e. en présence d'agents bifonctionnels, tels des diimides;
+ faire réagir les acides gras sous des formes réactives (plus réactives), i.e. faire réagir des halogénures (chlorures, bromures, iodures ...) d'acides gras, des anhydrides d'acides gras, des dérivés d'anhydride d'acides gras.

Selon ladite voie enzymatique, originale, faisant l'objet de la revendication de procédé 17 à 21, on couple les protéines aux chaînes grasses en présence d'une enzyme, généralement à une température comprise entre 30 et 70°C. Avantageusement ladite température est comprise entre 50 et 60°C. Avantageusement, l'enzyme intervenant est une acyltransférase. Selon trois variantes de cette voie enzymatique, ladite enzyme est une lipase, notamment choisie parmi les lipases de Mucor miehei, de pancréas de porc, de Rhizopus arrhizus, de Candida, de Bacillus et d'Apergillus ou une protéase, consistant notamment en la papaïne ou une amidase. Une telle réaction enzymatique assure, comme les réactions chimiques rappelées ci-dessus, le greffage de chaînes grasses sur les protéines. Lesdites chaînes grasses, lorsqu'il s'agit d'acides gras, peuvent intervenir sous forme d'esters (y compris sous forme d'esters de glycérides). L'enzyme présente dans le milieu réactionnel assure, dans un premier temps, la trans-estérification.

La réaction mise en oeuvre, chimique ou enzymatique, assure le couplage en générant des liaisons covalentes esters et/ou amides. On a vu que ledit couplage fait également intervenir des liaisons ioniques, des liaisons hydrogènes, des liaisons de type Van der Waals...

La réaction est avantageusement mise en oeuvre avec une activité de l'eau du milieu réactionnel (a_{w}) comprise entre 0,2 et 1 et avantageusement entre 0,3 et 0,7.

Les procédés de préparation des compositions de l'invention, compositions notamment cosmétiques, pharmaceutiques et alimentaires renfermant des complexes hydrolipidiques font également partie de l'invention. Ils consistent principalement à mélanger l'ingrédient actif à un excipient convenable. On a vu que ledit principe actif pouvait présenter des propriétés émulsionnantes. Ceci peut se révéler particulièrement intéressant. On peut ainsi limiter voire annuler l'intervention de tout agent émulsionnant synthétique.

L'invention est illustrée, sous ses différents aspects, par les exemples ci-après.

Tous les pourcentages indiqués sont des pourcentages en poids, sauf indication contraire.

### Exemple 1:

### Préparation d'un complexe protéine de soja-acide laurique (C12)

1 660 g d'acide laurique, de pureté égale à 99 %, sont chauffés jusqu'à 60°C dans un réacteur sous azote. Après fusion des chaînes d'acide laurique et obtention d'une huile incolore, 470 g d'un isolat de soja (masse moléculaire moyenne : 50 000 D), contenant au minimum 96 % de protéines natives, sont alors ajoutés en pluie fine dans le réacteur sous agitation mécanique modérée.

Après obtention d'une suspension homogène, 300 g de lipase extraite à partir de Mucor miehei, immobilisée sur résine échangeuse d'anion macroporeuse (nom commercial : Lipozyme^{®} de la société Novo) sont ajoutés au réacteur. L'ensemble est conservé 15 jours à 60°C dans un réacteur clos sous agitation mécanique modérée.

Après 15 jours de réaction, le complexe est filtré à 90°C afin d'éliminer l'enzyme. Le complexe ainsi obtenu est mis sous forme de copeaux lors de son refroidissement.

Après analyses, il s'avère que ce complexe est constitué de protéines lipophilisées dont environ 16 % des fonctions amines libres (latérales et terminales) ont été greffées par des acides gras (l'acide laurique).

Ce complexe se présente sous la forme d'une poudre de copeaux de couleur beige, d'odeur caractéristique. Il peut être utilisé dans une formulation cosmétique à 3 % et grâce à l'amphiphilicité apportée par le greffage, il est possible de l'incorporer dans les phases aqueuse et/ou huileuse d'une préparation cosmétique.

### Exemple 2 :

### Préparation d'un complexe protéine de soja-acides stéarique (C18) et palmitique (C16)

270 g d'acide stéarique et 180 g d'acide palmitique, chacun de pureté supérieure à 90 %, sont placés dans 1 000 ml de tertiobutanol, puis chauffés jusqu'à 60°C dans un réacteur sous azote. Après fusion des chaînes d'acides et obtention d'une huile incolore, 150 g d'un isolat de soja (masse moléculaire moyenne : 50 000 D), contenant au minimum 96 % de protéines natives, sont alors ajoutés en pluie fine dans le réacteur sous agitation mécanique modérée.

Après obtention d'une suspension homogène, 45 g de lipase extraite à partir de Rhizopus arrhizus sont ajoutés au réacteur. L'ensemble est conservé 21 jours à 55°C dans un réacteur clos sous agitation mécanique modérée.

Après 21 jours de réaction, le complexe est porté à 90°C pendant 20 min afin d'inactiver toute activité enzymatique résiduelle. Le tertiobutanol est alors éliminé par distillation sous pression réduite. Le complexe ainsi obtenu est mis sous forme de copeaux lors de son refroidissement.

Après analyses, il s'avère que ce complexe est constitué de protéines lipophilisées dont environ 21 % des fonctions amines latérales ont été greffées par des acides gras.

Ce complexe se présente sous la forme d'une poudre de copeaux de couleur beige, d'odeur caractéristique. Il peut être utilisé dans une formulation cosmétique à 3 %, et grâce à l'amphiphilicité apportée par le greffage, il est possible de l'incorporer dans les phases aqueuse et/ou huileuse d'une préparation cosmétique.

### Exemple 3 :

### Préparation d'un complexe protéine de blé-acides stéarique (C18) et palmitique (C16)

On procède comme décrit à l'exemple 2 en substituant au 150 g de l'isolat de soja, 150 g d'un atomisat obtenu à partir d'une solution de protéine de blé (masse moléculaire moyenne : 100 000 D). On obtient un complexe du type de celui décrit à l'exemple 2 (copeaux de couleur beige, d'odeur caractéristique).

### Exemple 4 :

### Préparation d'un complexe protéine d'amande-acides stéarique (C18) et palmitique (C16)

300 g d'acide stéarique et 140 g d'acide palmitique, chacun de pureté supérieure à 90 %, sont placés dans 1 000 ml d'isopropanol, puis chauffés jusqu'à 60°C dans un réacteur sous azote. Après fusion des chaînes d'acides et obtention d'une phase huileuse homogène, 150 g d'un lyophilisat obtenu à partir d'une solution de protéine d'amande (masse moléculaire moyenne : 30 000 D), sont alors ajoutés en pluie fine dans le réacteur sous agitation mécanique modérée.

Après obtention d'une suspension homogène, 35 g de lipase extraite à partir de Rhizopus arrhizus sont ajoutés au réacteur. L'ensemble est conservé 12 jours à 55°C dans un réacteur clos sous agitation mécanique modérée.

Après 12 jours de réaction, le complexe est porté à 90°C pendant 20 min afin d'inactiver toute activité enzymatique résiduelle. L'isopropanol est alors éliminé par distillation sous pression réduite. Le complexe ainsi obtenu est mis sous forme de copeaux lors de son refroidissement.

Ce complexe se présente sous la forme d'une poudre de copeaux de couleur beige, d'odeur caractéristique. Il peut être utilisé dans une formulation cosmétique à 3 %, et grâce à l'amphiphilicité apportée par le greffage, il est possible de l'incorporer dans les phases aqueuse et/ou huileuse d'une préparation cosmétique.

### Exemple 5:

On procède comme décrit aux exemples 2 à 4 mais le solvant intervenant est choisi parmi l'hexane, le chloroforme, le cyclohexane, le chlorométhane, le dichlorométhane, le trichlorométhane, l'éther éthylique, le méthyltertiobutyléther, ou un mélange de ces solvants.

### Exemple 6:

On procède comme décrit dans les exemples précédents mais en faisant varier la nature de l'enzyme utilisée : lipase de Mucor miehei, de pancréas de porc, de Rhizopus arrhizus, de Candida, de Bacillus, d'Aspergillus ou autres acyl-transférases.

### Exemple 7 :

On procède comme décrit dans les exemples précédents mais en faisant varier la nature de la protéine intervenante : protéine de blé, d'avoine, de maïs, d'amande, de soja.

### Exemple 8 :

On procède comme décrit dans les exemples précédents mais en faisant varier les paramètres de la réaction de couplage ci-après :
- la proportion entre chaînes grasses et protéines (ou polypeptides) ;
- la température de réaction (entre la température ambiante et 80°C) ;
- le temps de réaction (de 30 minutes à 21 jours).

### Exemple 9 :

On procède comme décrit dans les exemples précédents mais en faisant varier la nature de l'acide gras intervenant : l'acide heptanoïque (C7), l'acide octanoïque (C8), l'acide décanoïque (C10), l'acide laurique (C12), l'acide myristique (C14), l'acide palmitique (C16), l'acide stéarique (C18), l'acide ricinoléiquee (C18), l'acide oléique (C18), l'acide linoléique (C18), l'acide linolénique (C18), d'autres acides gras à chaînes plus courtes ou plus longues, saturés, insaturés ou polyinsaturés, utilisés purs ou en mélanges.

### Exemple 10 :

On procède comme décrit dans les exemples précédents mais les acides gras qui sont placés à réagir sur les protéines sont sous formes d'esters, et la lipase utilisée réalise une réaction de trans-estérification et/ou de trans-acylation. Ainsi, le linoléate d'éthyle, l'oléate d'isopropyle et le linolénate de glycérol ainsi que différentes huiles végétales sous forme de triglycérides (dont l'huile de coprah) ont été utilisés pour fournir la chaîne grasse qui va ensuite venir se greffer sur la protéine.

### Exemple 11:

On procède comme décrit dans les exemples 1 à 9 mais les acides gras qui sont placés à réagir sur les protéines sont sous une forme réactive, du type halogénure d'acide ou anhydride d'acide. Dans ce cas, la réaction peut être effectuée dans l'eau, à température ambiante et ne nécessite pas de lipase. De telles réactions sont explicitement décrites aux exemples 17 et 18.

### Exemple 12 :

On procède comme décrit dans les exemples 1 à 8 mais les chaînes grasses qui sont placées à réagir sur les protéines sont sous une forme alcool (formation de liaisons esters avec les fonctions acide carboxylique de la protéine) ou sous une forme amine (formation de liaisons amides avec les fonctions acide carboxylique de la protéine). De telles réactions sont explicitement décrites aux exemples 14 et 16 ci-après.

### Exemple 13 :

On procède comme décrit dans les exemples 2 à 12 mais sans utiliser de solvant.

### Exemple 14:

### Préparation d'un complexe protéine de soja-décylalcool (C10)

300 g de décylalcool, de pureté supérieure à 90 %, sont chauffés jusqu'à 60°C, en présence de 670 ml de tertiobutanol dans un réacteur sous azote. Après fusion des chaînes de décylalcool et obtention d'une huile incolore, 100 g d'un isolat de soja (masse moléculaire moyenne : 50 000 D), contenant au minimum 96 % de protéines natives, sont alors ajoutés en pluie fine dans le réacteur sous agitation mécanique modérée.

Après obtention d'une suspension homogène, 30 g de lipase extraite à partir de Rhizopus arrhizus sont ajoutés au réacteur. L'ensemble est conservé 10 jours à 60°C dans un réacteur clos sous agitation mécanique modérée.

Après 10 jours de réaction, le complexe est porté à 90°C pendant 20 min afin d'inactiver toute activité enzymatique résiduelle. Le tertiobutanol est alors éliminé par distillation sous pression réduite. Le complexe ainsi obtenu est mis sous forme de copeaux lors de son refroidissement.

Après analyses, il s'avère que ce complexe est constitué de protéines lipophilisées par des chaînes grasses, et environ 12 % des alcools gras ont été couplés à la protéine (à l'aide de liaisons covalentes comme des fonctions esters, mais également à l'aide de fonctions ioniques).

Ce complexe se présente sous la forme d'une poudre de copeaux de couleur beige, d'odeur caractéristique. Il peut être utilisé dans une formulation cosmétique à 3 %, et grâce à l'amphiphilicité apportée par le greffage, il est possible de l'incorporer dans les phases aqueuse et/ou huileuse d'une préparation cosmétique.

### Exemple 15:

On procède comme décrit dans les exemples précédents mais les complexes formés sont dispersés en phase aqueuse et leur pH est ajusté de façon à être compatible avec des formulations cosmétiques, à l'aide d'une base minérale ou organique. Les complexes ainsi obtenus peuvent ensuite être séchés par atomisation, lyophilisation ou séchage sous vide.

### Exemple 16:

### Préparation d'un complexe protéine de soja-laurylamine (C12)

300 g de laurylamine, de pureté supérieure à 90 %, sont chauffés jusqu'à 60°C, en présence de 670 ml de tertiobutanol dans un réacteur sous azote. Après fusion des chaînes de laurylamine et obtention d'une huile jaunâtre, 100 g d'un isolat de soja (masse moléculaire moyenne : 50 000 D), contenant au minimum 96 % de protéines natives, sont alors ajoutés en pluie fine dans le réacteur sous agitation mécanique modérée.

Après obtention d'une suspension homogène, 30 g de lipase extraite à partir de Rhizopus arrhizus sont ajoutés au réacteur. L'ensemble est conservé 10 jours à 60°C dans un réacteur clos sous agitation mécanique modérée.

Après 10 jours de réaction, le complexe est porté à 90°C pendant 20 min afin d'inactiver toute activité enzymatique résiduelle. Le tertiobutanol est alors éliminé par distillation sous pression réduite. 4 000 ml d'eau sont alors ajoutés au complexe et l'ensemble est porté à 70°C sous agitation modérée ; puis environ 1,5 moles de HCl (sous forme de solution 6N) sont ajoutées lentement au mélange réactionnel de façon à obtenir un pH compris entre 5,0 et 7,0. Le complexe ainsi obtenu est ensuite séché par lyophilisation.

Après analyses, il s'avère que ce complexe est constitué de protéines lipophilisées par des chaînes grasses, et environ 11 % des amines grasses ont été couplées à la protéine (à l'aide de liaisons covalentes comme des fonctions amides, mais également à l'aide de fonctions ioniques).

Ce complexe se présente sous la forme d'une poudre fine de couleur beige, d'odeur caractéristique. Il peut être utilisé dans une formulation cosmétique à 3 %, et grâce à l'amphiphilicité apportée par le greffage, il est possible de l'incorporer dans les phases aqueuse et/ou huileuse d'une préparation cosmétique.

### Exemple 17 :

### Préparation d'un complexe protéine de blé-acides stéarique (C18) et palmitique (C16)

100 g de protéine de blé soluble et de haute masse moléculaire moyenne (100 000 D) extraite du gluten de blé sont placés dans 5 000 ml d'eau déminéralisée. Le milieu réactionnel est ajusté à pH 11 par une solution d'hydroxyde de sodium (NaOH, 12N). Sous très forte agitation type Ultraturrax ou Silverson (10 000 à 20 000 rpm), 300 g d'un mélange de chlorures d'acides stéarique et palmitique sont alors lentement ajoutés. Le pH passe en quelques dizaines de minutes d'une valeur de 11 à une valeur proche de 1, lorsqu'aucun tampon n'est ajouté au milieu réactionnel. Après un temps de réaction d'une heure environ à température ambiante, l'ensemble est neutralisé jusqu'à un pH voisin de 7,0 par une solution d'hydroxyde de sodium (NaOH, 12N). L'ensemble est alors lyophilisé puis éventuellement stérilisé par rayons gamma ou béta. Le produit se présente sous la forme d'une poudre pulvérulente blanche, qui peut aussi bien être placée dans les phases aqueuses que dans les phases huileuses de préparations, cosmétiques par exemple. Une partie des acides gras a réagi avec la protéine pour former des liaisons amides et esters, et une partie qui n'a pas réagi se trouve toutefois sous forme étroitement complexée par des liaisons hydrogènes et par des liaisons de type Van der Waals, à la protéine.

### Exemple 18:

### Préparation d'un complexe protéine d'amande-acides stéarique (C18) et palmitique (C16)

On met en oeuvre la même technique de greffage que dans l'exemple 17 ci-dessus, mais une protéine d'amande, de masse moléculaire moyenne voisine de 30 000 D est utilisée à la place de la protéine de blé. La réaction est effectuée en régulant le pH à 11 par ajout de carbonate de sodium. Le complexe issu de ce greffage est conservé sous forme liquide et renferme 5 % de matière sèche ; 0,2 % de parabènes et 0,5 % de gomme de xanthane sont alors ajoutés. Le complexe ainsi formé est commercialisé sous la forme de cette solution ainsi décrite.

### Exemple 19:

On procède comme décrit dans les exemples 17 et 18 mais en opérant à des températures comprises entre 20 et 100°C. Les réactions de greffage réalisées à très hautes températures donnent des rendements plus importants mais donnent lieu à des dégradations modérées à sévères des protéines utilisées.

La liaison convalente qui résulte de cette réaction fournit des liaisons amides, mais d'autres liaisons caractéristiques sont présentes entre l'amine grasse et le polypeptide, comme des liaisons ioniques et des liaisons de type Van der Waals.

### Tolérance et toxicité :

Des études d'irritation cutanée et oculaire (réalisées selon des protocoles en accord avec les directives OCDE N°404 (12 mai 1981) et N° 405 (24 février 1987), ont été réalisées avec plusieurs des produits obtenus selon les exemples ci-dessus (exemples 1 à 19) sous forme de solutions à 10 %. Dans tous les cas, les produits sont apparus comme "non irritants" (n'ont provoqué aucun signe d'irritation cutanée ou oculaire), et ont été extrêmement bien tolérés.

De même, l'administration par voie orale de doses maximales de 5 g de ces produits par kilogramme de poids corporel n'a provoqué aucune toxicité (tests effectués selon un protocole en accord avec la ligne directrice de l'OCDE concernant l'étude de la toxicité par voie orale (N° 401 (24 février 1987).

Par ailleurs, des tests de sensibilisation selon le protocole de Magnusson et Kligman ont été réalisés avec ces produits en solution dans l'eau à 10 % et ces produits ont été classés parmi les produits ne possédant pas de propriété sensibilisante.

### Exemple 20 :

### Formulation anti-âge, restructurante CC591

| Phase | Produits | Noms INCI | Quantités (%) |
|---|---|---|---|
| A | Brij 72 | Steareth 2 | 3 |
| | Brij 721 | Steareth 21 | 2 |
| | Isostéaryl Isostéarate | Isostearyl Isostearate | 4 |
| | Huile de noyaux d'abricot | Apricot Kernel Oil | 4 |
| | Huile de safran | Safflower Oil | 2 |
| | Diméthicone 556 | Dimethicone 556 | 2 |
| | Crodacol CS50 | Cetostearyl Alcool | 3 |
| | | | |
| B | Eau | Water | qsp 100 |
| | Glycérine | Glycerin | 5 |
| | Produit de l'invention préparé selon l'exemple 2 | | 6 |
| C | Phénonip^{®} | Phenoxyethanol | 0,5 |
| | | Methylparaben | |
| | | Ethylparaben | |
| | | Propylparaben | |
| | | Butylparaben | |
| | | | |
| D | Propylène glycol | Propylene Glycol | 0,5 |
| | | | |
| | Parfum | | 0,3 |
| | Alpha tocophérol | Alpha Tocopherol | 0,05 |

On chauffe séparément les phases A et B à 75°C, sous agitation modérée. On ajuste le pH de la phase B à la valeur de pH souhaitée. On verse A dans B sous agitation très violente (du type Silverson ou Ultraturrax), puis on laisse chuter la température sous agitation lente. A 30°C, on rajoute les composants des phases C et D.

### Exemple 21:

### Formulation anti-âge, visage CC585

| Phase | Produits | Noms INCI | Quantités (%) |
|---|---|---|---|
| A | Isostéaryl Isostéarate | Isostearyl Isostearate | 4 |
| | Huile de Carthame | Safflower Oil | 4 |
| | Cétiol J600 | Oleyl Erucate | 2 |
| | Diméthicone 556 | Dimethicone | 5 |
| | Crodacol CS50 | Cetostearyl Alcool | 3 |
| | Produit dé l'invention réalisé selon l'exemple 17 : | | 3 |
| B | Glycérine | Glycerin | 5 |
| | Eau | Water | qsp 100 |
| | | | |
| C | Phénonip^{®} | Phenoxyethanol | 0,5 |
| | | Methylparaben | |
| | | Ethylparaben | |
| | | Propylparaben | |
| | | Butylparaben | |
| | Propylène glycol | Propylene Glycol | 0,5 |
| D | Parfum | Perfume | 0,3 |

On chauffe séparément les phases A et B à 75°C, sous agitation modérée. Le pH de la formule est conditionné dans ce cas par le pH du produit de l'invention. On verse A dans B sous agitation très violente (du type Silverson ou Ultraturrax), puis on laisse chuter la température sous agitation lente. A 30°C, on rajoute les composants des phases C et D. Si nécessaire, on ajuste la préparation au pH désiré, à l'aide d'acide lactique par exemple.

### Exemple 22.

### Formulation peaux sèches, visage

| Phase | Produits | Noms INCI | Quantités (%) |
|---|---|---|---|
| A | Huile de bourrache | Borrage Oil | 2 |
| | Huile de Carthame | Safflower Oil | 4 |
| | Myritol 318 | Caprylic/Capric triglyceride | 6 |
| | Crodacol CS50 | Cetostearyl Alcool | 3 |
| B | Glycérine | Glycerin | 5 |
| | Eau | Water | qsp 100 |
| | Produit de l'invention | selon l'exemple 2 | 4 |
| C | Phénonip® | Phenoxyethanol | 0,5 |
| | | Methylparaben | |
| | | Ethylparaben | |
| | | Propylparaben | |
| | | Butylparaben | |
| | Propylène glycol | Propylene Glycol | 0,5 |
| D | Parfum | Perfume | 0,3 |

On chauffe séparément les phases A et B à 75°C, sous agitation modérée. Le pH de la phase B est ajusté au pH de la formulation désiré. On verse A dans B sous agitation très violente (du type Silverson ou Ultraturrax), puis on laisse chuter la température sous agitation lente. A 30°C, on rajoute les composants des phases C et D. Si nécessaire, on ajuste la préparation au pH désiré, à l'aide d'acide lactique par exemple.

### Exemple 23 :

### Fomulation shampooing familial

| Phase | Produits | Noms INCI | Quantités (%) |
|---|---|---|---|
| A | Texapon N40^{®} (Henkel) | Sodium Laureth Sulfate | 40 |
| | Comperlan KD^{®} (Henkel) | Cocamide DEA | 2 |
| B | Produit de l'invention selon | l'exemple 17 | 0,3 |
| | Eau | Water | qsp 100 |
| | Chlorure de sodium | Sodium Chloride | 1,5 |
| C | Phénonip^{®} | Phenoxyethanol | 0,5 |
| | | Methylparaben | |
| | | Ethylparaben | |
| | | Propylparaben | |
| | | Butylparaben | |
| | Propylène glycol | Propylene Glycol | 0,5 |

On chauffe séparément la phase B à 75°C, sous agitation modérée. Le pH de ladite phase B est ajusté au pH de la formulation désiré. On verse B, dans A à 20°C, sous agitation très lente puis on laisse chuter la température. A 30°C, on rajoute la phase C.

### Exemple 24:

### Formulation shampooing doux

| Phase | Produits | Noms INCI | Quantités (%) |
|---|---|---|---|
| A | Tween 20^{®} (ICI) | Polysorbate 20 | 10 |
| | TegoBetaine L7^{®} (Goldschmidt) | Cocamidopropyl Betaine | 10 |
| | Atlas G1821^{®}(ICI) | PEG-150 Distearate | 3 |
| B | Produit de l'invention selon l'exemple 18 | | 0,5 |
| | Eau | Water | qsp 100 |
| C | Phénonip^{®} | Phenoxyethanol | 0,5 |
| | | Methylparaben | |
| | | Ethylparaben | |
| | | Propylparaben | |
| | | Butylparaben | |
| | Propylène glycol | Propylene Glycol | 0,5 |

On chauffe séparément la phase B à 75°C, sous agitation modérée. Le pH de ladite phase B est ajusté au pH de la formulation désiré. On homogénéise A sous agitation à 20°C. On verse B, dans A à 20°C, sous agitation très lente puis on laisse chuter la température. A 30°C, on rajoute la phase C.

### Exemple 25 :

### Formulation shampooing nacré

| Phase | Produits | Noms INCI | Quantités (%) |
|---|---|---|---|
| A | Texapon N40^{®} (Henkel) | Sodium Laureth Sulfate | 40 |
| | Comperlan KD^{®} (Henkel) | Cocamide DEA | 2 |
| | Euperlan PK771^{®} (Henkel) | Glycol Distearate (and) | 4 |
| | | Sodium Laureth Sulfate | |
| | | (and) Cocamide MEA (and) | |
| | | Laureth-10 | |
| | | | |
| B | Produit de l'invention selon | l'exemple 16 | 0,5 |
| | Eau | Water | qsp 100 |
| | Chlorure de sodium | Sodium Chloride | 1,5 |
| C | Phénonip^{®} | Phenoxyethanol | 0,5 |
| | | Methylparaben | |
| | | Ethylparaben | |
| | | Propylparaben | |
| | | Butylparaben | |
| | Propylène glycol | Propylene Glycol | 0,5 |

On chauffe séparément la phase B à 75°C, sous agitation modérée. Le pH de la phase B est ajusté au pH de la formulation désiré. On homogénéise B à 20°C. On verse B, dans A à 20°C, sous agitation très lente puis on laisse chuter la température. A 30°C, on rajoute la phase C.

### Exemple 26:

### Utilisation d'un complexe protéine de blé-acides stéarique et palmitique dans des applications cosmétiques "restructurantes" et permettant de lutter contre les effets du vieillissement

Les complexes réalisés selon les exemples 3 (par voie enzymatique) et 17 (par voie chimique) ont été testés pour leur capacité à lisser le micro-relief cutané. En effet, l'aspect extérieur de la peau est révélateur de son état général, et les mailles formées par le réseau micro-dépressionnaire cutané ont tendance à s'agrandir et à se creuser au cours du vieillissement. D'autres facteurs extérieurs peuvent également contribuer à ce phénomène, comme par exemple l'utilisation de détergents. Cette désorganisation du micro-relief est le signe d'une altération de la couche cornée et de sa fonction de barrière protectrice naturelle. Elle donne un aspect rugueux et un toucher rèche au tégument, puis conduit à une déshydratation prononcée de celui-ci.

L'activité restructurante de ces complexes a été étudiée après une destruction importante du réseau micro-dépressionnaire, obtenue par une agression chimique du revêtement cutané à l'aide d'une solution aqueuse contenant 10 % de détergent (lauryl sulfate de sodium). Les essais ont été réalisés sur la face externe des deux mains de 10 volontaires. Chaque main a été lavée 4 fois par jour pendant 30 secondes, à 1/2 h d'intervalle, pendant 4 jours avec cette solution de détergent. Une des mains a reçu à l'issue de ces traitements, chaque jour, un traitement réalisé à partir d'une solution contenant 3 % du complexe réalisé selon l'exemple 3 ou 17 de l'invention. Chaque jour, la réparation cutanée a été évaluée comparativement aux zones témoins, agressées et non traitées, par observation directe de la surface cutanée au stéréo microscope, et par étude des stripping. L'efficacité du complexe a été comparée à celle de la protéine de blé utilisée pour la réalisation dudit complexe ; les pouvoirs filmogènes et adoucissants de la protéine de blé étant bien connus.

Les deux produits (protéine et protéine complexée) améliorent nettement et d'une façon quasi-évidente l'aspect visuel de la surface cornée ; cependant, seul le complexe protéine de blé-acides gras selon l'invention, présente un pouvoir restructurant extrêmement important (voisin de 90 %), qui ne se limite pas à freiner l'agression due au détergent, mais qui permet une régénération de -. l'ensemble du tégument. Ainsi, les peaux traitées sont fréquemment en meilleur état après dégradation et application de la solution aqueuse du complexe préparé à l'exemple 3 ou 17, qu'avant tout traitement.

Ainsi, il est possible d'affirmer que le complexe protéine de blé-chaînes palmitique et stéarique est un actif cosmétique "régénérant", capable d'équilibrer et d'harmoniser la cohésion de l'épiderme.

### Exemple 27:

### Utilisation d'un complexe protéine d'amande-acides stéarique et palmitique dans des applications cosmétiques permettant d'apaiser les agressions cutanées liées aux érythèmes solaires

Les complexes réalisés selon les exemples 4 (par voie enzymatique) et 18 (par voie chimique) ont été testés pour leur capacité à réduire et calmer les érythèmes solaires ; en effet, les coups de soleil répétés favorisent d'une part une altération des mécanismes biochimiques de la peau, par la destruction des lipides des membranes cellulaires, par la fragmentation des macromolécules biologiques indispensables aux réparations cutanées, et d'autre part l'accélération du vieillissement cutané ; la conjonction de ces deux phénomènes pouvant parfois se traduire par l'apparition de cancers cutanés.

Le pouvoir anti-érythémateux du complexe protéine d'amande-acides stéarique et palmitique a été étudié chez le cobaye dont la réaction érythèmale est bien corrélée à celle de l'homme. Les irradiations des animaux ont été réalisées à l'aide de 2 lampes Philipps TL 40W/12 émettant entre 280 et 340 nm avec un pic à 315 nm. Placé à 3 % au sein d'une émulsion (voir composition A ci-dessous), le complexe réalisé selon l'exemple 4 ou 18 a été testé comparativement à une émulsion placebo (voir composition C ci-dessous) et à une émulsion contenant le polypeptide d'amande utilisé dans le complexe, sous forme non complexé (voir composition B ci-dessous). Dans chaque cas, 0,25 ml de produit a été administré immédiatement après irradiation, puis 2, 5 et 24 h après l'exposition. L'érythème étant évalué selon une cotation visuelle de 0 (pas d'érythème) à 4 (érythème intense), l'effet réducteur d'érythème a été mesuré 2, 5, 24 et 48 h après l'irradiation, comparativement aux zones témoins irradiées mais non traitées. Les résultats ont ensuite été exprimés en pourcentage d'inhibition de l'érythème.

| Phase | Ingrédients | A | B | C |
|---|---|---|---|---|
| A | Complexe décrit dans l'exemple 4 ou 18 | | 3 0 | 0 |
| | Polypeptide d'amande | 0 | 0,75 | 0 |
| | Eau | qsp 100 | qsp 100 | qsp 100 |
| | | | | |
| B | Huile de noyaux d'abricot | 5 | 5 | 5 |
| | Isostéaryl d'isostéarate | 5 | 5 | 5 |
| | Erucate d'oléyle | 2 | 2 | 2 |
| | Alcool cétostéarylique | 3 | 3 | 3 |
| | | | | |
| C | Huile de silicone | 2 | 2 | 2 |
| | Parabènes | 0,2 | 0,2 | 0,2 |

Préparation des compositions : Les phases A et B sont chauffées séparément à 75°C. Après une bonne homogénéisation, B est versé dans A sous agitation très intense, puis l'ensemble est porté à refroidir sous agitation lente. A 30°C, la phase C est alors ajoutée.
**Résultats** : Des érythèmes modérés ont été réalisés sur les cobayes. Ils correspondent à des érythèmes d'indice 1,5 à 24 h. L'effet adoucissant procuré par l'application d'une émulsion cosmétique de l'art antérieur (préparation placebo C) sur ces érythèmes, bien que sensible, reste insuffisant pour combattre efficacement le développement de la réaction inflammatoire. Par contre, le pouvoir anti-érythémateux du complexe (préparation A) est immédiat (inhibition de l'érythème d'environ 30 %, 2 h après l'application) et durable tout au long des traitements (inhibition de 45 %, 45 % et 65 % après respectivement 5, 24 et 48 h). La préparation réalisée avec le polypeptide d'amande non complexé (préparation B) ne permet pas non plus d'obtenir de tels résultats.

D'autres résultats ont été obtenus sur des érythèmes provoqués beaucoup plus prononcés (érythèmes d'indice 2 à 24 h). Dans ce cas, l'efficacité de la préparation. A est encore plus prononcée par rapport aux efficacité obtenues avec les autres préparations.

Il est donc possible d'affirmer que le complexe protéine d'amande-chaînes stéarique et palmitique, utilisé dans une formulation cosmétique, est un actif qui permet de réparer durablement les effets destructeurs des érythèmes solaires modérés voire forts, observés lors d'expositions solaires prolongées.

## Revendications

1. Composition cosmétique renfermant à titre d'ingrédient actif au moins une protéine, **caractérisée en ce qu'**elle renferme, au moins en partie, ladite protéine sous la forme d'un complexe amphiphile résultant de la réaction, à une température comprise entre la température ambiante et 80°C, entre d'une part au moins une protéine végétale native dont la masse moléculaire moyenne est supérieure ou égale à 10 000 Dalton et d'autre part au moins une chaîne grasse dont le nombre d'atomes de carbone est compris entre 4 et 30, choisie parmi les acides gras, les alcools gras, les amines grasses et leurs dérivés à l'exclusion de l'acide undécylénique; le rapport pondéral des réactifs protéine(s)/chaîne(s) grasse(s) variant de 1/1 à 1/10.

2. Composition pharmaceutique renfermant à titre d'ingrédient actif au moins une protéine, **caractérisée en ce qu'**elle renferme, au moins en partie, ladite protéine sous la forme d'un complexe amphiphile résultant de la réaction, à une température comprise entre la température ambiante et 80°C, entre d'une part au moins une protéine végétale native dont la masse moléculaire moyenne est supérieure ou égale à 10 000 Dalton et d'autre part au moins une chaîne grasse dont le nombre d'atomes de carbone est compris entre 4 et 30, choisie parmi les acides gras, les alcools gras, les amines grasses et leurs dérivés à l'exclusion de l'acide undécylénique; le rapport pondéral des réactifs protéine(s)/chaîne(s) grasse(s) variant de 1/1 à 1/10.

3. Composition selon la revendication 1 ou 2, **caractérisée en ce que** la protéine végétale a une masse moléculaire moyenne comprise entre 10 000 et 100 000 Dalton.

4. Composition selon l'une des revendications 1 à 3, **caractérisée en ce que** la chaîne grasse a un nombre d'atomes de carbone compris entre 6 et 20.

5. Composition selon l'une quelconque des revendication 1 à 4, **caractérisée en ce que** le complexe amphiphile consiste en le produit de ladite réaction en mélange avec les chaînes grasses n'ayant pas réagi.

6. Composition selon l'une quelconque des revendications 1 à 5, **caractérisée en ce que** la protéine végétale native est choisie parmi les protéines de blé, de maïs, de coton, de lupin, de pois, de fève, d'amande, de féverole, de soja, de tournesol, de luzerne, d'avoine.

7. Composition selon l'une quelconque des revendications 1 à 6, **caractérisée en ce que** la chaîne grasse est choisie parmi les acides gras heptanoïque, octanoïque, décanoïque, laurique, myristique, palmitique, stéarique, ricinoléique, oléique, linoléique, linolénique; les alcools gras et amines grasses correspondants; les dérivés desdits acides gras, alcools et amines; et leurs mélanges.

8. Composition selon l'une des revendications 1 à 7, **caractérisée en ce que** le complexe amphiphile précité comprend une protéine de blé soluble possédant une masse moléculaire moyenne de 100 000 Dalton, sur laquelle des chaînes d'acide stéarique et palmitique ont été greffées.

9. Composition selon l'une des revendications 1 à 6, **caractérisée en ce que** le complexe amphiphile précité comprend une protéine d'amande soluble possédant une masse moléculaire moyenne de 30 000 Dalton sur laquelle des chaînes d'acide stéarique et palmitique ont été greffées.

10. Composition selon la revendication 9, **caractérisée en ce que** la composition cosmétique est présente sous forme d'une formulation solaire ou après solaire.

11. Composition selon l'une des revendications 1 à 8, **caractérisée en ce que** le complexe amphiphile précité comprend une protéine de laminaire insoluble possédant une masse moléculaire moyenne de 10 000 Dalton sur laquelle des chaînes d'acide caprylique ont été greffées.

12. Composition selon l'une des revendications 1 à 11, **caractérisée en ce que** le rapport pondéral des réactifs protéine(s)/chaîne(s) grasse(s) varie de 1/3 à 1/5.

13. Composition selon l'une des revendications 1 à 12, **caractérisée en ce que** la protéine a une masse moléculaire moyenne comprise entre 20 000 et 300 000 Dalton.

14. Composition selon l'une des revendications 1 à 11, **caractérisée en ce que** la composition comprend de 0,01 à 40 % en poids, du complexe amphiphile précité.

15. Composition selon l'une des revendications 1 à 14, **caractérisée en ce que** la composition comprend de 0,1 à 10 % en poids, du complexe amphiphile précité.

16. Utilisation d'une protéine sous la forme d'un complexe amphiphile résultant de la réaction, à une température comprise entre la température ambiante et 80°C, entre d'une part au moins une protéine végétale native dont la masse moléculaire moyenne est supérieure ou égale à 10 000 Dalton et d'autre part au moins une chaîne grasse dont le nombre d'atomes de carbone est compris entre 4 et 30, choisie parmi les acides gras, les alcools gras, les amines grasses et leurs dérivés à l'exclusion de l'acide undécylénique; le rapport pondéral des réactifs protéine(s)/chaîne(s) grasse(s) variant de 1/1 à 1/10, à titre d'ingrédient actif pour la fabrication d'une composition cosmétique.

17. Utilisation d'une protéine sous la forme d'un complexe amphiphile résultant de la réaction, à une température comprise entre la température ambiante et 80°C, entre d'une part au moins une protéine végétale native dont la masse moléculaire moyenne est supérieure ou égale à 10 000 Dalton et d'autre part au moins une chaîne grasse dont le nombre d'atomes de carbone est compris entre 4 et 30, choisie parmi les acides gras, les alcools gras, les amines grasses et leurs dérivés à l'exclusion de l'acide undécylénique; le rapport pondéral des réactifs protéine(s) /chaîne(s) grasse(s) variant de 1/1 à 1/10, à titre d'ingrédient actif pour la fabrication d'une composition pharmaceutique.

18. Utilisation selon la revendication 16 ou 17, **caractérisée en ce que** le complexe amphiphile est tel que défini à l'une quelconque des revendications 3 à 15.

19. Procédé de soin cosmétique, **caractérisé en ce qu'**il comprend l'application sur la peau, ou les cheveux, d'une composition cosmétique telle que définie à l'une quelconque des revendications 1, 3 à 15.

20. Procédé de préparation d'un complexe amphiphile tel que défini à l'une quelconque des revendications 1 à 13, **caractérisée en ce qu'**il comprend la réaction dite de couplage, par voie enzymatique, à une température comprise entre 30 et 70 °C si nécessaire en milieu aqueux ou solvant entre au moins une protéine végétale native ayant une masse moléculaire moyenne supérieure ou égale à 10 000 Dalton et au moins une chaîne grasse ayant dont le nombre d'atome de carbone est compris entre 4 et 30, choisie parmi les acides gras, les alcools gras, les amines grasses et leurs dérivés à l'exclusion de l'acide undécylénique; lesdits réactifs intervenant dans un rapport pondéral protéine(s)/chaîne(s) grasse(s) variant de 1/1 à 1/10; et ledit couplage enzymatique est mis en oeuvre avec une enzyme choisie parmi le groupe consistant d'une lipase, telle une lipase de Mucor miehei, de pancréas de porc, de Rhizopus arrhizus, de Candida, de Bacillus ou d'Aspergillus, ou d'une protéase telle la papaïne ou d'une amidase.

21. Procédé selon la revendication 20, **caractérisé en ce que** l'activité de l'eau (aw) du milieu réactionnel est comprise entre 0,2 et 1.

22. Procédé selon la revendication 20 ou 21, **caractérisé en ce que** les acides gras interviennent sous forme d'ester, y compris d'esters de glycérides.

23. Procédé selon l'une quelconque des revendications 20 à 22, **caractérisé en ce que** le couplage enzymatique est mis en oeuvre à une température comprise entre 50 et 60°C.

24. Procédé selon l'une quelconque des revendications 20 à 23, **caractérisé en ce qu'**on prépare un complexe amphiphile choisi parmi le groupe consistant d'un complexe de protéine de soja, de masse moléculaire moyenne 50 000 Dalton et d'acide laurique avec une lipase extraite à partir de Mucor miehei; un complexe de protéine de soja de masse moléculaire moyenne de 50 000 Dalton - acides stéarique et palmitique préparé avec une lipase extraite à partir de Rhizopus arrhizus; un complexe de protéine de blé de masse moléculaire moyenne de 100 000 Dalton - acides stéarique et palmitique avec une lipase extraite à partir de Rhizopus arrhizus, un complexe de protéine d'amande soluble de masse moléculaire moyenne de 30 000 Dalton - acides stéarique et palmitique avec une lipase extraite à partir de Rhizopus arrhizus; un complexe de protéine de soja de masse moléculaire moyenne de 50 000 Dalton -décylalcool avec une lipase extraite à partir de Rhizopus arrhizus; ou un complexe de protéine de soja de masse moléculaire moyenne de 50 000 Dalton - laurylamine avec une lipase extraite à partir de Rhizopus arrhizus.

25. Procédé selon l'une des revendications 20 à 24, **caractérisé en ce que** le rapport pondéral protéine(s)/chaîne(s) grasse(s) varie de 1/3 à 1/5.

26. Procédé selon l'une des revendications 21 à 25, **caractérisé en ce que** l'activité de l'eau (aw) du milieu réactionnel est comprise entre 0,3 et 0,7.

27. Composition comprenant un complexe amphiphile résultant de la réaction, à une température comprise entre la température ambiante et 80°C, entre d'une part au moins une protéine végétale native, dont la masse moléculaire moyenne est supérieure ou égale à 10 000 Dalton, et d'autre part au moins une chaîne grasse dont le nombre d'atomes de carbone est compris entre 4 et 30, choisi parmi les acides gras, les alcools gras, les amines grasses et leurs dérivés à l'exclusion de l'acide undécylénique; le rapport pondéral des réactifs protéine(s)/chaîne(s) grasse(s) variant de 1/1 à 1/10, ladite protéine végétale étant choisie parmi le groupe consistant en des protéines de blé, de maïs, de coton, de lupin, de poids, de fève, d'amande, de fèverole, de soja, de tournesol, de luzerne, d'avoine, ledit complexe consistant en le produit de ladite réaction, en mélange avec les chaînes grasses n'ayant pas réagi.

28. Composition selon la revendication 27, **caractérisée en ce que** la chaîne grasse est choisie parmi les acides gras heptanoïque, octanoïque, décanoïque, laurique, myristique, palmitique, stéarique, ricinoléique, oléique, linoléique, linolénique; les alcools gras et amines grasses correspondants; les dérivés desdits acides gras, alcools et amines ; et leurs mélanges.

29. Composition selon la revendication 27 ou 28, **caractérisée en ce qu'**il s'agit d'un complexe d'un isolat de soja ayant une masse moléculaire moyenne de 50 000 Dalton et d'un acide gras tel que défini à la revendication 28.

30. Composition selon la revendication 27 ou 28, **caractérisée en ce qu'**il s'agit d'un complexe protéine de blé ayant une masse moléculaire moyenne de 100 000 Dalton avec un acide gras tel que défini à la revendication 28 , en particulier un mélange d'acides stéarique et palmitique.

31. Composition selon la revendication 27 ou 28, **caractérisée en ce qu'**il s'agit d'un complexe protéine d'amande de masse moléculaire moyenne de 30 000 Dalton et d'un acide gras tel que défini à la revendication 28, en particulier un mélange d'acides stéarique et palmitique.

32. Composition selon la revendication 27, **caractérisée en ce qu'**il s'agit d'un complexe de protéine de soja de masse moléculaire moyenne de 50 000 Dalton et de décylalcool ou de lauryl amine.

33. Utilisation d'une composition telle que définie à l'une quelconque des revendications 27 à 32 pour la fabrication d'une composition cosmétique ou pour la fabrication d'une composition pharmaceutique.

34. Utilisation selon la revendication 33 d'un complexe protéine de blé ayant une masse moléculaire moyenne de 100 000 Dalton - acides stéarique et palmitique pour la fabrication d'une composition cosmétique pour la restructuration cutanée et la lutte contre les effets du vieillissement.

35. Utilisation selon la revendication 33 d'un complexe protéine d'amande ayant une masse moléculaire moyenne de 30 000 Dalton - acides stéarique et palmitique pour la fabrication d'une composition cosmétique permettant d'apaiser les agressions cutanées liées aux érythèmes solaires.

36. Utilisation selon la revendication 33 d'une protéine de laminaire insoluble, possédant une masse moléculaire moyenne de 10 000 Dalton, sur laquelle des chaînes d'acide caprylique ont été greffées, pour la fabrication d'une composition ayant la propriété d'inhiber un certain nombre de microorganisme pour un effet anti-acné, effet anti-pelliculaire, effet anti-odeur corporelle, conservateur naturel.

## Claims

1. Cosmetic composition containing as active ingredient at least one protein, the composition being **characterised in that** it contains, at least in part, said protein in the form of an amphiphilic complex resulting from the reaction, at a temperature between ambient temperature and 80°C, between, firstly at least one native plant protein whose average molecular mass is greater than or equal to 10,000 Daltons, and secondly at least one fatty chain whose carbon atom number is between 4 and 30, selected from fatty acids, fatty alcohols, fatty amines and derivatives thereof with the exception of undecylenic acid; the protein/fatty chain reagents weight ratio ranging from 1/1 to 1/10 .

2. Pharmaceutical composition containing as active ingredient at least one protein, the composition being **characterised in that** it contains, at least in part, said protein in the form of an amphiphilic complex resulting from the reaction, at a temperature between ambient temperature and 80°C, between, firstly at least one native plant protein whose average molecular mass is greater than or equal to 10,000 Daltons, and secondly at least one fatty chain whose carbon atom number is between 4 and 30, selected from fatty acids, fatty alcohols, fatty amines and derivatives thereof with the exception of undecylenic acid; the protein/fatty chain reagents weight ratio ranging from 1/1 to 1/10 .

3. Composition according to claim 1 or claim 2, **characterised in that** the plant protein has an average molecular mass between 10,000 and 100,000 Daltons.

4. Composition according to one of claims 1 to 3, **characterised in that** the fatty chain has a carbon atom number between 6 and 20.

5. Composition according to any one of claims 1 to 4, **characterised in that** the amphiphilic complex consists of the product of said reaction in admixture with the unreacted fatty chains.

6. Composition according to any one of claims 1 to 5, **characterised in that** the native plant protein is selected from the wheat, maize, cotton, lupin, pea, bean, almond, horse bean, soya, sunflower, alfalfa, oat proteins.

7. Composition according to any one of claims 1 to 6, **characterised in that** the fatty chain is selected from the heptanoic, octanoic, decanoic, lauric, myristic, palmitic, stearic, ricinoleic, oleic, linoleic, linolenic fatty acids; the corresponding fatty alcohols and fatty amines; the derivatives of said fatty acids, alcohols and amines; and mixtures thereof.

8. Composition according to one of claims 1 to 7, **characterised in that** the aforesaid amphiphilic complex comprises a soluble wheat protein having an average molecular mass of 100,000 Daltons onto which chains of stearic and palmitic acids have been grafted.

9. Composition according to one of claims 1 to 6, **characterised in that** the aforesaid amphiphilic complex comprises a soluble almond protein having an average molecular mass of 30,000 Daltons onto which chains of stearic and palmitic acids have been grafted.

10. Composition according to claim 9, **characterised in that** the cosmetic composition is present in the form of a sun or after-sun formulation.

11. Composition according to one of claims 1 to 8, **characterised in that** the aforesaid amphiphilic complex comprises an insoluble laminaria protein having an average molecular mass of 10,000 Daltons onto which chains of caprylic acid have been grafted.

12. Composition according to one of claims 1 to 11, **characterised in that** the protein/fatty chain reagents weight ratio ranges from 1/3 to 1/5.

13. Composition according to one of claims 1 to 12, **characterised in that** the protein has an average molecular mass between 20,000 and 300,000 Daltons.

14. Composition according to one of claims 1 to 11, **characterised in that** the composition contains from 0.01 to 40% by weight of aforesaid amphiphilic complex.

15. Composition according to one of claims 1 to 14, **characterised in that** the composition contains from 0.1 to 10% by weight of aforesaid amphiphilic complex.

16. Use of a protein in the form of an amphiphilic complex resulting from the reaction, at a temperature between ambient temperature and 80°C, between firstly at least one native plant protein whose average molecular mass is greater than or equal to 10,000 Daltons and secondly, at least one fatty chain whose carbon atom number is between 4 and 30, selected from fatty acids, fatty alcohols, fatty amines and derivatives thereof with the exception of undecylenic acid; the protein/fatty chain reagents weight ratio ranging from 1/1 to 1/10, as active ingredient for preparing a cosmetic composition.

17. Use of a protein in the form of an amphiphilic complex resulting from the reaction, at a temperature between ambient temperature and 80°C, between firstly at least one native plant protein whose average molecular mass is greater than or equal to 10,000 Daltons and secondly, at least one fatty chain whose carbon atom number is between 4 and 30, selected from fatty acids, fatty alcohols, fatty amines and derivatives thereof with the exception of undecylenic acid; the protein/fatty chain reagents weight ratio ranging from 1/1 to 1/10, as active ingredient for preparing a pharmaceutical composition.

18. Use according to claim 16 or claim 17, **characterised in that** the amphiphilic complex is as defined in any one of claims 3 to 15.

19. Method of cosmetic care, **characterised in that** it comprises the application on the skin or hair, of a cosmetic composition as defined in any one of claims 1, 3 to 15.

20. Method of preparing an amphiphilic complex as defined in any one of claims 1 to 13, **characterised in that** it comprises said "coupling" reaction, via the enzymatic route, at a temperature between 30 and 70°C if necessary in an aqueous or solvent medium between at least one native plant protein whose average molecular mass is greater than or equal to 10,000 Daltons and at least one fatty chain whose carbon atom number is between 4 and 30, selected from fatty acids, fatty alcohols, fatty amines and derivatives thereof with the exception of undecylenic acid; said reagents intervening in a protein/fatty chain weight ratio ranging from 1/1 to 1/10; and said enzymatic coupling is carried out with an enzyme selected from the group consisting of a lipase, such as a Mucor miehei lipase, a pig pancreas lipase, a Rhizopus arrhizus lipase, a Candida lipase, a Bacillus lipase, an Aspergillus lipase, or a protease, such as papaine or an amidase.

21. Method according to claim 20, **characterised in that** the water activity (aw) of the reaction medium is between 0.2 and 1.

22. Method according to claim 20 or claim 21, **characterised in that** the fatty acids intervene as esters, including esters of glycerides.

23. Method according to any one of claims 20 to 22, **characterised in that** enzymatic coupling is carried out at a temperature between 50 and 60°C.

24. Method according to any one of claims 20 to 23, **characterised in that** an amphiphilic complex is prepared which is selected from the group consisting of a soya protein complex of average molecular mass of 50,000 Daltons and of lauric acid with a lipase extracted from Mucor miehei; a soya protein complex of average molecular mass of 50,000 Daltons - stearic and palmitic acids prepared with a lipase extracted from Rhizopus arrhizus; a wheat protein complex of average molecular mass of 100,000 Daltons - stearic and palmitic acids with a lipase extracted from Rhizopus arrhizus, a soluble almond protein complex of average molecular mass of 30,000 Daltons - stearic and palmitic acids with a lipase extracted from Rhizopus arrhizus; a soya protein complex of average molecular mass of 50,000 Daltons - decylalcohol with a lipase extracted from Rhizopus arrhizus; or a soya protein complex of average molecular mass of 50,000 Daltons - laurylamine with a lipase extracted from Rhizopus arrhizus.

25. Method according to one of claims 20 to 24, **characterised in that** the protein/fatty chain weight ratio ranges from 1/3 to 1/5.

26. Method according to one of claims 21 to 25, **characterised in that** the water activity (aw) of the reaction medium lies between 0.3 and 0.7.

27. A composition comprising an amphiphilic complex resulting from the reaction, at a temperature between ambient temperature and 80°C, between firstly at least one native plant protein whose average molecular mass is greater than or equal to 10,000 Daltons and secondly, at least one fatty chain whose carbon atom number is between 4 and 30, selected from fatty acids, fatty alcohols, fatty amines and derivatives thereof with the exception of undecylenic acid; the protein/fatty chain reagents weight ratio ranging from 1/1 to 1/10, said plant protein being selected from the group consisting of the wheat, maize, cotton, lupin, pea, bean, almond, horse bean, soya, sunflower, alfalfa, oat proteins, said complex consisting of the product of said reaction in admixture with the unreacted fatty chains.

28. Composition according to claim 27, **characterised in that** the fatty chain is selected from the heptanoic, octanoic, decanoic, lauric, myristic, palmitic, stearic, ricinoleic, oleic, linoleic, linolenic fatty acids; the corresponding fatty alcohols and fatty amines; the derivatives of said fatty acids, alcohols and amines; and mixtures thereof.

29. Composition according to claim 27 or claim 28, **characterised in that** it consists of a soya isolate complex of average molecular mass of 50,000 Daltons and of a fatty acid as defined in claim 28.

30. Composition according to claim 27 or claim 28, **characterised in that** it consists of a wheat protein complex of average molecular mass of 100,000 Daltons with a acid as in claim 28, in particular a mixture of stearic and palmitic acids.

31. Composition according to claim 27 or claim 28, **characterised in that** it consists of an almond protein complex of average molecular mass of 30,000 Daltons and of a fatty acid as defined in claim 28, in particular a mixture of stearic and palmitic acids.

32. Composition according to claim 27, **characterised in that** it consists of a soya protein complex of average molecular mass of 50,000 Daltons and of decylalcohol or laurylamine.

33. Use of a composition as defined in any one of claims 27 to 32 for preparing a cosmetic composition or for preparing a pharmaceutical composition.

34. Use according to claim 33 of a wheat protein complex having an average molecular mass of 100,000 Daltons - stearic and palmitic acids for preparing a cosmetic composition for skin restructuration and combating against skin aging effects.

35. Use according to claim 33 of an almond protein complex having an average molecular mass of 30,000 Daltons - stearic and palmitic acids for preparing a cosmetic composition which allows soothing skin aggression linked to solar erythema.

36. Use according to claim 33 of an insoluble laminaria protein having an average molecular mass of 10,000 Daltons onto which chains of caprylic acid have been grafted, for preparing a composition having the property of inhibiting a certain number of micro-organisms, for an anti-acne effect, an anti-dandruff effect, an anti-body odour effect, natural preservative.

## Patentansprüche

1. Kosmetische Zusammensetzung, die als Wirkstoff wenigstens ein Protein umfaßt, **dadurch gekennzeichnet, daß** sie wenigstens teilweise, das Protein in Form eines amphiphilen Komplexes umfaßt, der resultiert aus der Reaktion bei einer Temperatur zwischen der Umgebungstemperatur und 80 °C unter einerseits wenigstens einem nativen Pflanzenprotein, dessen mittlere Molekularmasse größer oder gleich 10.000 Dalton ist, und andererseits wenigstens einer Fettkette, deren Anzahl von Kohlenstoffatomen zwischen 4 und 30 liegt, gewählt unter den Fettsäuren, den Fettalkoholen, den Fettaminen und deren Derivaten unter Ausschluß von Undecylensäure, wobei das Gewichtsverhältnis der Reagenzien Protein(e)/Fettkette(n) von 1/1 bis 1/10 variiert.

2. Pharmazeutische Zusammensetzung, die als Wirkstoff wenigstens ein Protein umfaßt, **dadurch gekennzeichnet, daß** sie, wenigstens teilweise, das Protein in Form eines amphiphilen Komplexes umfaßt, der resultiert aus der Reaktion bei einer Temperatur zwischen der Umgebungstemperatur und 80 °C unter einerseits wenigstens einem nativen Pflanzenprotein, dessen mittlere Molekularmasse größer oder gleich 10.000 Dalton ist und andererseits wenigstens einer Fettkette, deren Anzahl von Kohlenstoffatomen zwischen 4 und 30 liegt, gewählt unter den Fettsäuren, den Fettalkoholen, den Fettaminen und deren Derivaten unter Ausschluß von Undecylensäure, wobei das Gewichtsverhältnis der Reagenzien Protein(e)/Fettkette(n) von 1/1 bis 1/10 variiert.

3. Zusammensetzung nach Anspruch 1 oder 2, **dadurch gekennzeichnet, daß** das Pflanzenprotein eine mittlere Molekularmasse zwischen 10.000 und 100.000 Dalton hat.

4. Zusammensetzung nach einem der Ansprüche 1 bis 3, **dadurch gekennzeichnet, daß** die Fettkette eine Anzahl von Kohlenstoffatomen zwischen 6 und 20 hat.

5. Zusammensetzung nach einem der Ansprüche 1 bis 4, **dadurch gekennzeichnet, daß** der amphiphile Komplex aus dem Reaktionsprodukt im Gemisch mit den Fettketten besteht, die nicht reagiert haben.

6. Zusammensetzung nach einem der Ansprüche 1 bis 5, **dadurch gekennzeichnet, daß** das native Pflanzenprotein gewählt wird unter den Proteinen von Weizen, Mais, Baumwolle, Lupine, Erbse, Bohne, Mandel, Pferdebohne, Soja, Sonnenblume, Luzerne, Hafer.

7. Zusammensetzung nach einem der Ansprüche 1 bis 6, **dadurch gekennzeichnet, daß** die Fettkette gewählt wird unter den Heptan-, Oktan-, Dekan-, Lauryl-, Myristin-, Palmitin-, Stearin-, Ricinol-, Öl-, Linol-, Linolenfettsäuren; den entsprechenden Fettalkoholen und Fettaminen; den Derivaten dieser Fettsäuren, Alkohole und Amine und deren Gemischen.

8. Zusammensetzung nach einem der Ansprüche 1 bis 7, **dadurch gekennzeichnet, daß** der oben genannte amphiphile Komplex ein lösliches Weizenprotein umfaßt, das eine mittlere Molekularmasse von 100.000 Dalton aufweist, auf welches Stearin- und Palmitinsäureketten gepfropft worden sind.

9. Zusammensetzung nach einem der Ansprüche 1 bis 6, **dadurch gekennzeichnet, daß** der oben genannte amphiphile Komplex ein lösliches Mandelprotein umfaßt, das eine mittlere Molekularmasse von 30.000 Dalton aufweist, auf welches Stearin- und Palmitinsäureketten gepfropft worden sind.

10. Zusammensetzung nach Anspruch 9, **dadurch gekennzeichnet, daß** die kosmetische Zusammensetzung in Form einer Sonnenschutz- oder Apres-Soleil-Sonnenschutzformulierung vorliegt.

11. Zusammensetzung nach einem der Ansprüche 1 bis 8, **dadurch gekennzeichnet, daß** der oben genannte amphiphile Komplex ein unlösliches Laminariaprotein umfaßt, das eine mittlere Molekularmasse von 10.000 Dalton aufweist, auf welches Caprylsäureketten gepfropft worden sind.

12. Zusammensetzung nach einem der Ansprüche 1 bis 11, **dadurch gekennzeichnet, daß** das Gewichtsverhältnis der Reagenzien Protein(e)/Fettkette(n) von 1/3 bis 1/5 variiert.

13. Zusammensetzung nach einem der Ansprüche 1 bis 12, **dadurch gekennzeichnet, daß** das Protein eine mittlere Molekularmasse zwischen 20.000 und 300.000 Dalton hat.

14. Zusammensetzung nach einem der Ansprüche 1 bis 11, **dadurch gekennzeichnet, daß** die Zusammensetzung 0,01 bis 40 Gewichtsprozent des oben genannten amphiphilen Komplexes umfaßt.

15. Zusammensetzung nach einem der Ansprüche 1 bis 14, **dadurch gekennzeichnet, daß** die Zusammensetzung 0,1 bis 10 Gewichtsprozent des oben genannten amphiphilen Komplexes umfaßt.

16. Verwendung eines Proteins in Form eines amphiphilen Komplexes, der resultiert aus der Reaktion bei einer Temperatur zwischen der Umgebungstemperatur und 80 °C unter einerseits wenigstens einem nativen Pflanzenprotein, dessen mittlere Molekularmasse größer oder gleich 10.000 Dalton ist und andererseits wenigstens einer Fettkette, deren Anzahl von Kohlenstoffatomen zwischen 4 und 30 liegt, gewählt unter den Fettsäuren, den Fettalkoholen, den Fettaminen und deren Derivaten unter Ausschluß von Undecylensäure, wobei das Gewichtsverhältnis der Reagenzien Protein(e)/Fettkette(n) von 1/1 bis 1/10 variiert, als Wirkstoff für die Herstellung einer kosmetischen Zusammensetzung.

17. Verwendung eines Proteins in Form eines amphiphilen Komplexes, der resultiert aus der Reaktion bei einer Temperatur zwischen der Umgebungstemperatur und 80 °C unter einerseits wenigstens einem nativen Pflanzenprotein, dessen mittlere Molekularmasse größer oder gleich 10.000 ist und andererseits wenigstens einer Fettkette, deren von Kohlenstoffatomen zwischen 4 und 30 liegt, gewählt unter den Fettsäuren, den Fettalkoholen, den Fettaminen und deren Derivaten unter Ausschluß von Undecylensäure, wobei das Gewichtsverhältnis der Reagenzien Protein(e)/Fettkette(n) von 1/1 bis 1/10 variiert, als Wirkstoff für die Herstellung einer pharmazeutischen Zusammensetzung.

18. Verwendung nach Anspruch 16 oder 17, **dadurch gekennzeichnet, daß** der amphiphile Komplex so wie bei einem der Ansprüche 3 bis 15 definiert ist.

19. Verfahren zur kosmetischen Pflege, **dadurch gekennzeichnet, daß** es die Anwendung auf die Haut oder die Haare von einer kosmetischen Zusammensetzung wie definiert in einem der Ansprüche 1, 3 bis 15 umfaßt.

20. Verfahren zur Herstellung eines amphiphilen Komplexes wie definiert in einem der Ansprüche 1 bis 13, **dadurch gekennzeichnet, daß** es die Kopplung genannte Reaktion auf enzymatischem Wege bei einer Temperatur zwischen 30 und 70 °C, wenn notwendig im wäßrigen Medium oder Lösungsmittelmedium unter wenigstens einem nativen Pflanzenprotein mit einer mittleren Molekularmasse größer oder gleich 10.000 Dalton und wenigstens einer Fettkette umfaßt, deren Anzahl von Kohlenstoffatomen zwischen 4 und 30 liegt, gewählt unter den Fettsäuren, den Fettalkoholen, den Fettaminen und deren Derivaten unter Ausschluß von Undecylensäure; wobei die Reagenzien in einem Gewichtsverhältnis Protein(e)/Fettkette(n) einwirken, das von 1/1 bis 1/10 variiert, und die enzymatische Kopplung mit einem Enzym ausgeführt wird, das gewählt ist aus der Gruppe, die besteht aus einer Lipase wie einer Lipase von Mucor miehei, Schweinepankreas, Rhizopus Arrhizus, Candida, Bacillus oder Aspergillus oder eine Protease wie Papain oder eine Amidase.

21. Verfahren nach Anspruch 20, **dadurch gekennzeichnet, daß** die Wasseraktivität (aw) des Reaktionsmediums zwischen 0,2 und 1 liegt.

22. Verfahren nach Anspruch 20 oder 21, **dadurch gekennzeichnet, daß** die Fettsäuren in Form eines Esters einwirken, einschließlich der Glyceridester.

23. Verfahren nach einem der Ansprüche 20 bis 22, **dadurch gekennzeichnet, daß** die enzymatische Kopplung bei einer Temperatur zwischen 50 und 60 °C ausgeführt wird.

24. Verfahren nach einem der Ansprüche 20 bis 23, **dadurch gekennzeichnet, daß** man einen amphiphilen Komplex herstellt, der gewählt ist, aus der Gruppe, die besteht aus einem Komplex aus Sojaprotein mit 50.000 Dalton mittlerer Molekularmasse und Laurylsäure mit einer Lipase, die extrahiert ist aus Mucor miehei, einem Komplex aus Sojaprotein mit mittlerer Molekularmasse von 50.000 Dalton - Stearin- und Palmitinsäuren, hergestellt mit einer Lipase, die extrahiert ist aus Rhizopus Arrhizus, einem Komplex aus Weizenprotein mit mittlerer molekularer Masse von 100.000 Dalton - Stearin- und Palmitinsäuren mit einer Lipase, die extrahiert ist aus Rhizopus Arrhizus, einem Komplex aus löslichem Mandelprotein mit mittlerer molekularer Masse von 30.000 Dalton - Stearin- und Palmitinsäuren mit einer Lipase, die extrahiert ist aus Rhizopus Arrhizus, einem Komplex aus Sojaprotein mit mittlerer molekularer Masse von 50.000 Dalton - Decylalkohol mit einer Lipase, die extrahiert ist aus Rhizopus Arrhizus, oder einem Komplex aus Sojaprotein mit mittlerer molekularer Masse von 50.000 Dalton - Laurylamin mit einer Lipase, die extrahiert ist aus Rhizopus Arrhizus.

25. Verfahren nach einem der Ansprüche 20 bis 24, **dadurch gekennzeichnet, daß** das Gewichtsverhältnis Protein(e)/Fettkette(n) von 1/3 bis 1/5 variiert.

26. Verfahren nach einem der Ansprüche 21 bis 25, **dadurch gekennzeichnet, daß** die Wasseraktivität (aw) des Reaktionsmediums zwischen 0,3 und 0,7 liegt.

27. Zusammensetzung, die einen amphiphilen Komplex umfaßt, der resultiert aus der Reaktion bei einer Temperatur zwischen der Umgebungstemperatur und 80 °C unter einerseits wenigstens einem nativen Pflanzenprotein, dessen mittlere molekulare Masse größer oder gleich 10.000 Dalton ist, und andererseits wenigstens einer Fettkette, deren Anzahl von Kohlenstoffatomen zwischen 4 und 30 liegt, gewählt unter den Fettsäuren, den Fettalkoholen, den Fettaminen und deren Derivaten unter Ausschluß von Undecylensäure, wobei das der Reagenzien Protein(e)/Fettkette(n) von 1/1 bis 1/10 variiert, wobei das Pflanzenprotein gewählt ist aus der Gruppe, die besteht aus den Proteinen von Weizen, Mais, Baumwolle, Lupine, Erbse, Bohne, Mandel, Pferdebohne, Soja, Sonnenblume, Luzerne, Hafer, wobei der Komplex besteht aus dem Reaktionsprodukt im Gemisch mit den Fettketten, die nicht reagiert haben.

28. Zusammensetzung nach Anspruch 27, **dadurch gekennzeichnet, daß** die Fettkette gewählt wird unter den Heptan-, Oktan-, Dekan-, Lauryl-, Myristin-, Palmitin-, Stearin-, Ricinol-, Öl-, Linol-, Linolenfettsäuren ; den entsprechenden Fettalkoholen und Fettaminen; den Derivaten dieser Fettsäuren, Alkohole und Amine und deren Gemischen.

29. Zusammensetzung nach Anspruch 27 oder 28, **dadurch gekennzeichnet, daß** es sich um einen Komplex eines Isolats aus Soja mit einer mittleren Molekularmasse von 50.000 Dalton und einer Fettsäure wie in Anspruch 28 definiert handelt.

30. Zusammensetzung nach Anspruch 27 oder 28, **dadurch gekennzeichnet, daß** es sich um einen Komplex aus Weizenprotein mit einer mittleren Molekularmasse von 100.000 Dalton mit einer Fettsäure wie in Anspruch 28 definiert, insbesondere ein Gemisch von Stearin- und Palmitinsäuren handelt.

31. Zusammensetzung nach Anspruch 27 oder 28, **dadurch gekennzeichnet, daß** es sich um einen Komplex aus Mandelprotein mit einer mittleren Molekularmasse von 30.000 Dalton und mit einer Fettsäure wie in Anspruch 28 definiert handelt, insbesondere ein Gemisch von Stearin- und Palmitinsäuren.

32. Zusammensetzung nach Anspruch 27, **dadurch gekennzeichnet, daß** es sich um einen Komplex aus Sojaprotein mit mittlerer Molekularmasse von 50.000 Dalton und Decylalkohol oder Laurylamin handelt.

33. Verwendung einer Zusammensetzung wie in einem der Ansprüche 27 bis 32 definiert für die Herstellung einer kosmetischen Zusammensetzung oder für die Herstellung einer pharmazeutischen Zusammensetzung.

34. Verwendung nach Anspruch 33 von einem Komplex aus mit einer mittleren von 100.000 Dalton - Stearin- oder Palmitinsäuren, für die Herstellung einer kosmetischen Zusammensetzung zur Hautrestrukturierung und der Alterungswirkungen.

35. Verwendung nach Anspruch 33 von einem Komplex aus Mandelprotein mit einer mittleren Molekularmasse von 30.000 Dalton - Stearin- und Palmitinsäuren, zur Herstellung einer kosmetischen Zusammensetzung, die es erlaubt, die Hautangriffe zu dämpfen, die mit Sonnenerythemen verbunden sind.

36. Verwendung nach Anspruch 33 von einem unlöslichen Blatttankprotein, das eine mittlere molekulare Masse von 10.000 Dalton aufweist, auf welches Caprylsäureketten gepfropft worden sind, zur Herstellung einer Zusammensetzung mit der Eigenschaft, eine bestimmte Anzahl von Mikroorganismen zu inhibieren für eine Anti-Aknewirkung, Anti-Schuppenwirkung, Anti-Körpergeruchswirkung, natürliche Konservierwirkung.
